# EUROPEAN PATENT SPECIFICATION

(11) **EP 0 281 626 B1**
(45) Date of publication and mention of the grant of the patent: **05.06.1996**
(21) Application number: 87907166.0
(22) Date of filing: 24.08.1987
(51) Int. Cl.: G01N 33/48, G01N 33/566, G01N 33/53, G01N 33/554

(54) **METHOD AND APPARATUS FOR MEASURING THE DEGREE OF REACTION BETWEEN A FOREIGN ENTITY AND A SUBJECT'S BLOOD CELLS**
VERFAHREN UND VORRICHTUNG ZUR FESTSTELLUNG DES REAKTIONSGRADES ZWISCHEN EINEM FREMDKÖRPER UND DEN BLUTZELLEN EINES PATIENTEN
PROCEDE ET APPAREIL DE MESURE DE LA REACTIVITE ENTRE UNE ENTITE ETRANGERE ET LES CELLULES SANGUINES D'UN PATIENT

(30) Priority: 28.08.1986 US 902313; 01.10.1986 US 913940
(43) Date of publication of application: 14.09.1988
(73) Proprietor: AMTL CORPORATION, Hollywood, FL 33020 (US)
(72) Inventor: Pasula, Mark J., c/o Signet Diagnostics Corp., Palm Beach Gardens, FL 33410 (US)
(74) Representative: Patentanwälte Wenzel & Kalkoff
(86) International application number: US8702237
(87) International publication number: WO8801743

(56) References cited:
- EP-A- 0 140 379
- US-A- 4 614 722

## Description

### BACKGROUND OF THE INVENTION

This invention relates to the field of cellular immune reactions; and, more particularly, to a method for the direct and/or indirect determination of the degree of reaction (if any) between a foreign entity and a subject's immune system for diagnosing a malady of the subject.

Diagnosing maladies is perhaps the single most important aspect of medicine. The key to diagnosing a malady is based on an understanding of the malady's cause.

Many illnesses and afflictions (i.e. maladies other than illnesses, such as an allergy or cancer) are caused by an individual's coming into contact with foreign entities, such as environmental chemicals, including known or possible carcinogens, other toxins or micro-organisms, including viruses and bacteria.

Every day, the human body is exposed to many types of foreign entities, and when so exposed, the body may ingest some foreign entities, by eating or breathing them in, or perhaps merely by touch. Once a foreign entity is ingested, the body identifies it either as being neutral, in which case the body does not react in any extraordinary fashion, or identifies it as being potentially harmful, in which case the body acts to defend itself.

If the foreign entity is in fact harmful, such as a carcinogen or a virus, it will cause certain ill effects to the individual, such as disease. However, even if the foreign entity is benign, and might not cause any untoward effects by itself, the reaction of the body to its identification as being harmful may have its own set of ill effects, such as an allergic reaction.

The body's defenses, primarily the immune system, and the manner in which they act to defend the body, have been greatly studied. In broad terms, the white blood cells (leukocytes) in an individual's blood act as the first line of defense against foreign entities classified as harmful. Once a foreign entity is identified as being harmful, besides the natural (innate) immune response, the body may produce specific antibodies which combine with the foreign entity and, in conjunction with the leukocytes, destroy the invading foreign entity.

I have observed three different steps in the response of the leukocytes to a foreign entity identified as harmful, after the antibodies and leukocytes combine.

First, the leukocyte increases in volume, to surround and enclose the foreign entity. This reaction is similar or identical to a phagocytosis reaction, in which a cell ingests a particle, Bellanti, J.A., Immunology III, W.B. Saunders Co. (1985), p. 16.

Second, after the foreign entity is enclosed by the leukocyte, the volume of the leukocyte decreases in a so-called complement reaction, i.e. the leukocyte develops a small defect in its cell membrane, and begins to extrude a portion of its cellular material, Bryant, N.J., Immunohematology, W.B. Saunders Co. (1982), pp. 54-55.

Finally, the leukocyte releases all of its cellular material, and breaks up its outer membranes. This reaction is similar to the degranulation of basophiles noticed in the presence of IgE mediated reactions, Bellanti, supra., p. 252.

I have recently discovered that some red blood cells and some platelets also participate in the body's immune response to an invading substance, undergoing reactions similar to those of the leukocytes. This phenomenon has not heretofore been recognized, and its discovery may assist in the understanding of the body's immune reactions.

Many theories exist as to the precise mechanism behind the operation of the immune system, i.e. how blood cells recognize and define harmful foreign entities, and how antibodies are produced, etc. No currently known theory, however, explains all aspects of the body's defensive reactions. In addition, no currently known theory is generally accepted as the sole basis for explaining the reactions.

This lack of an understanding of the immune system response has hampered efforts to devise a uniform and comprehensive diagnostic tool or method for the diagnosis of a wide spectrum of maladies. Since a comprehensive understanding of the mechanics of the immune response is lacking, there is no comprehensive understanding of how maladies may be recognized at an early stage.

Currently, diagnosis of a malady is more or less by a look-up method. A subject approaches a doctor and relates his symptoms. The doctor then matches those symptoms with the symptoms of known maladies, and attempts to cull a short list of possible causes from all of the conceivable causes. Based on this list, the doctor will perform tests to isolate the cause of the malady. If there is no positive test result, then a new series of tests will be performed, and this procedure continues until a positive result is attained. This may take a great deal of time and expense, and some of the tests performed may be discomforting or even painful for the subject.

This procedure is necessary because most tests are directed to specific symptoms of an illness. For example, an illness which affects kidney function may be indicated by an increased level of urea in the subject's bodily fluids. The test, then, for that kidney ailment, would be to check the level of urea in the subject's bodily fluids. Such tests do not identify the malady per se, but rather measure an expected bodily response to the malady's presence.

The look-up method has many drawbacks, however. First, it depends upon the subject's ability to recognize symptoms. If the subject has not started to feel the effects of the malady, then he may not know enough to tell the doctor of a minor symptom which would indicate a serious malady. It is for this reason that a wide battery of tests is often prescribed for a new subject, to ascertain to the extent possible what may be ailing that subject.

These tests may be time-consuming, expensive and even painful. Additionally, if the right tests are not called for, someone may be diagnosed as being in good health, but in fact have a massive tumor (for example) which has not yet begun to cause any visible symptoms. If a subject is suffering from more than one malady, the various symptoms may also mask or disguise each other, leading to a false diagnosis.

A different problem arises if two maladies have similar symptoms. A diagnosis based on symptoms may be unable to discern two completely different maladies having similar symptoms.

Furthermore, the subjectivity of an individual as to the experiencing or relating of certain symptoms may also come into play. For example, a slight headache may not cause an individual any great concern, but may in fact indicate a brain tumor.

An incorrect and possibly fatal diagnosis is always a serious concern to doctors, and there is a serious need for an objective test which may be used to diagnose a variety of maladies, without the possibility of masking or disguising of symptoms, and which may be performed effectively, relatively inexpensively, objectively, and quickly.

Further complicating this situation is the possibility that a particular malady may not cause any noticeable symptoms until it has become quite serious. Thus, the afflicted individual may not know enough to have tests performed.

The test described in my earlier Patent published as EP-A-0 140 379 assists in the diagnosis of allergies by a simple blood test. That disclosure, however, neither teaches nor suggests any applicability of the test disclosed therein beyond allergies, nor does it indicate anything beyond the application of a suspected allergen.

Furthermore, prior tests are not useful in diagnosing maladies for which there are no antibodies produced by the subject. If the introduction of the foreign entity to the body does not result in the production of antibodies specific to the foreign entity, then the body will not react to attack it and no reaction will take place. This is the case, for example, with respect to carcinogens. Since the body may not produce any natural antibodies specific to the carcinogen, the body may not act to attack it. Thus, a cancer caused by the carcinogen is free to develop unfettered, until it is of a size sufficient to be detected by other conventional means, such as by palpation or X-ray, No early detection is possible, however, until symptoms are evident. In many instances, the onset of symptoms means imminent death.

There is thus a need for an objective test which may be used to diagnose a wider variety of maladies, and which may be used to diagnose maladies caused by the ingestion of foreign entities for which no antibodies are produced by the subject. There is also a need for a method of diagnosis in which maladies may be diagnosed at a stage before the onset of externally observable symptoms.

There is therefore still a need for a test which may be used to assist in the diagnosis and treatment of maladies which affect the red blood cells or platelets of a subject.

### OBJECTS AND SUMMARY OF THE INVENTION

It is thus an object of the invention to provide a method for the objective determination of the degree of reaction between a foreign entity and the blood cells of a subject.

It is a further object of the invention to provide such an improved method where accuracy and reliability will not depend upon the subjective interpretation of symptoms by a subject or his physician.

It is another object of the invention to provide a comprehensive testing method which may be used for the diagnosis of a wide variety of maladies, without the need for a multiplicity of tests and procedures.

It is a further object of the invention to provide a method of diagnosis which is capable of diagnosing maladies caused by foreign entities for which the body of a subject produces no antibodies.

In accordance with these and other objects of the invention there is provided a method for diagnosing the presence or absence of a malady affecting the immune system of a subject, the method comprising the steps of:
counting a number of blood cells of a at least one type selected from the group consisting of red blood cells and platelets, in a first blood sample drawn from the subject; mixing a specific foreign entity known to provoke the malady or an antibody specific to the malady with a second blood sample drawn from the subject to form a mixture, and thereby allow blood cells of the second blood sample to react with the foreign entity or the antibody; counting a number of unreacted and reacted blood cells in the mixture; comparing the number of the blood cells counted in the first blood sample with the number of unreacted and reacted blood cells counted in the mixture, to determine if the blood cells in the second blood sample reacted with the foreign entity or the antibody, thereby diagnosing the presence or absence of the malady in the subject.

In accordance with a preferred embodiment of the invention, there is further provided a method of the type described above, wherein said first and second blood samples further include leukocytes and each of said first blood sample and said second blood sample have approximately equal distributions of blood cells therein, said method including: lysing at least a portion of said blood cells in said first blood sample, counting a number of leucocytes in said first blood sample, mixing a specific foreign entity known to provoke said malady or an antibody specific to said malady with said second blood sample, thereby producing a mixture, lysing a portion of said blood cells in said mixture, counting a number of leucocytes in said mixture, and comparing said number of leucocytes in said mixture with said number of said leucocytes in said first blood sample, thereby producing a further result, whereby the presence or absence of said malady may also be determined by said further result.

In accordance with a third embodiment of the invention, there is provided a method for diagnosing the presence or absence of a malady affecting the immune system of a subject which is indicated by the presence of a foreign entity, the method comprises the steps of: counting a number of leukocytes in a first blood sample drawn from said subject, mixing an antibody specific to said malady with a second blood sample drawn from said subject to form a mixture, and thereby allow leukocytes of said second blood sample to react with said antibody and said foreign entity if present, counting a number of unreacted and reacted leukocytes in said mixture, and comparing said number of said leukocytes counted in said first blood sample with said number of unreacted and reacted leukocytes counted in said mixture, to determine if said leukocytes in said second blood sample reacted with said antibody, thereby diagnosing the presence or absence of said malady in said subject.

In accordance with a fourth preferred embodiment of the invention there is provided a further method for diagnosing the presence or absence of a malady affecting the immune system of a subject which is indicated by the presence of a foreign entity, said method comprising the steps of: sizing a number of leukocytes in a first blood sample drawn from said subject, mixing an antibody specific to said malady with a second blood sample drawn from said subject to form a mixture, and thereby allow leukocytes present in said second blood sample to react with said antibody and said foreign entity if present, sizing a number of unreacted and reacted leukocytes in said mixture, and comparing said sized leukocytes of said first blood sample with said sized unreacted and reacted leukocytes of said mixture, to determine thereby if at least a portion of said leukocytes in said second blood sample reacted with said antibody, whereby the presence of said malady in said subject is indicated by a significant difference therebetween.

Further preferred embodiments of the invention are described in the dependent claims.

Briefly stated, the invention is directed to a method for the diagnosis of a malady in a subject by the observing of a degree of reaction between all blood cells: red blood cells, leukocytes and platelets; in the subject's blood with a foreign entity having a predetermined relationship with the malady being diagnosed. The test includes comparing amounts and sizes of red blood cells, leukocytes and/or platelets in a control sample and at least one test sample. The test sample includes a portion of the subject's blood and the foreign entity being tested.

The above, and other objects, features and advantages of the present invention will become apparent from the following description read in conjunction with the accompanying drawings, in which like reference numerals designate the same elements.

### BRIEF DESCRIPTION OF THE DRAWINGS

FIG. 1 is a block diagram showing the connection of the various components which make up the present apparatus; and
FIG. 2 is a flow chart showing the steps of the inventive method.

### DETAILED DESCRIPTION OF THE PREFERRED EMBODIMENT

My referenced application, describes a test for use in the diagnosis of allergies by measuring the degree of reaction between the leukocytes in a subject's blood with a suspected allergen. The disclosure of that application is herein incorporated by reference.

What is not evident from a reading of that application, however, is my discovery that many common maladies, and some less common maladies, cause reactions in a subject's blood similar to those resulting from allergies. It is my theory that the reactions of the body's immune system in responding to the identification of an allergen, i.e. a leukocyte engulfing the allergen, enlarging and then extruding its contents, is basically identical to the bodily reactions to the introduction of other foreign entities recognized as being harmful, such as a virus or even a carcinogen. In sum, it is my theory that most maladies may be diagnosed by the observation of the reactions of the body's immune system, specifically, the size-distribution of the leukocytes in two samples of a subject's blood: a control sample which has no foreign entity added thereto, and a test sample which has at least one foreign entity added thereto.

The basic method of the invention and apparatus disclosed herein is contained in my earlier application, but the breadth of its applicability is significantly wider than heretofore realized, in its reach beyond merely diagnosing maladies other than allergies.

What I have recently discovered, however, is that certain red blood cells and some platelets react to an invading foreign entity in the same fashion as do leukocytes, i.e. they combine with antibodies to react with foreign entities, causing the lysing of red cells. The discovery of this bodily process may, for example, assist in the diagnosis and treatment of anemias, which may be broadly defined as maladies in which a subject has a low red blood cell count.

For example, if a subject has a low red blood cell count due to an allergic reaction to certain foreign entities, then the avoidance of those foreign entities may lead to a normal red blood cell count. The testing for such a reaction by a subject's red blood cells may be performed along lines generally similar to those of my earlier tests.

Furthermore, similar results by testing platelets may be achieved for other maladies. More research is required for determining which types of blood cells are best used to test for certain maladies, but the discovery that all blood cells react in an allergic-type reaction to the presence of an invading foreign entity leads to the importance of testing all types of blood cells for reaction with the foreign entity. For ease of reference, the term "blood cells", as used herein is meant to include platelets, red blood cells and leukocytes, even though platelets are not generally considered to be blood cells for all purposes.

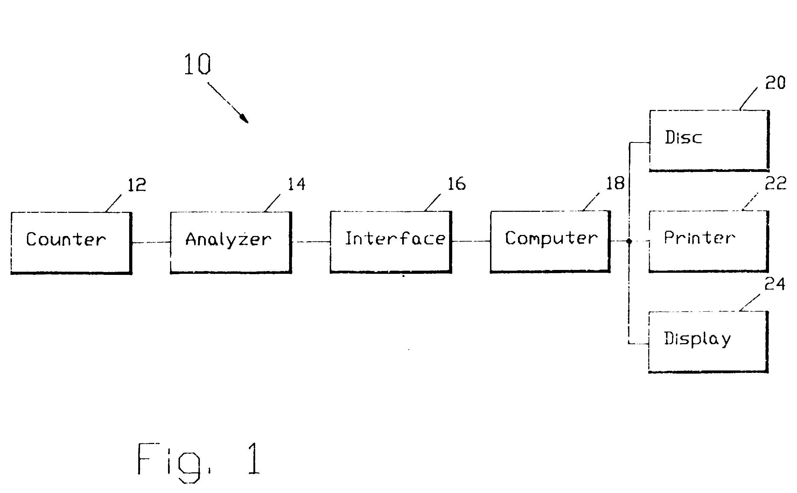

An apparatus for performing the tests of the inventive method, as shown generally at 10 in Fig. 1, comprises a counter 12, an analyzer 14, a computer interface 16 and a computer 18, each connected in series, and a disc 20,. a printer 22 and a video display 24, each connected to the output of computer 18.

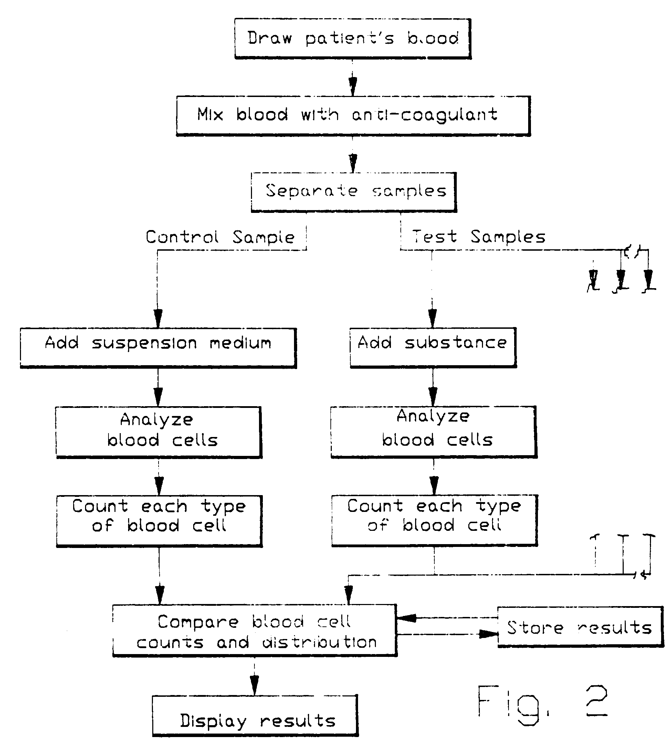

To commence the operation of the apparatus (generally described in the flow chart of Fig. 2) a blood sample is first drawn from the person being tested (subject). Preferably, 10ml of oxylated blood is drawn from the subject, using sodium citrate as an anti-coagulant (i.e. the blood is stored in blue top B-D brand vacutainers) to prevent clotting of the blood during the test. This single sample may be used to perform a wide variety of tests for a vast array of different maladies.

It is necessary to secure an even cellular distribution of blood cells over the entire volume of the blood sample, and so it is necessary to transfer blood from the vacutainer to an apparatus (not shown) which will have the capability of continuous flow circulation, in known fashion. Such a device will cause the cells of the sample to be distributed generally evenly over the entire sample.

It is preferred that a single blood sample drawn from the subject be used during a battery of tests for a number of foreign entities, and so the blood drawn from the subject is separated into a plurality of smaller samples for testing. The preferred method of separation is to place 100 microliters of the drawn blood into a receptacle containing an appropriate suspension medium, e.g. one-half ml of a balanced pH saline solution. The precise amount of the suspension medium used is not critical, except that the same amount thereof should be used throughout the test, i.e. in each sample of the same blood. This will ensure that measurements taken for the test and control samples may be directly compared by virtue of the fact that they both have a similar number of leukocytes and volume.

The 10ml of drawn blood prepared as described will be suitable for approximately 75 tests.

After separation of the drawn sample into the control and test samples, the foreign entities are introduced into the test samples in the form of solutions.

Suitable solutions may be purchased commercially from any one of a number of suppliers.
Alternatively, suitable solutions may be prepared by adding 100mg of a dried extract of that foreign entity to 10ml of Isoton II or III (distributed by Coulter), or any mixture thereof, or in 10ml of a suitable diluting solution. In many instances, injectable paragen-free, sterilized water will be suitable, in others perhaps alcohol. The particular diluting solution selected will depend upon the foreign entity being mixed, and the selection of an appropriate diluting solution is well within the knowledge of those of ordinary skill in the art. Once made, the mixture is allowed to stand for 24 hours at room temperature, and then filtered through a mesh capable of filtering solid particles.

Different solutions of foreign entities are introduced into the various test samples. It is preferred that one foreign entity be introduced into each test sample, but it is contemplated that, in some embodiments, each test sample may have a wide variety of foreign entities introduced thereto, so that a single drawn blood sample, which may otherwise be usable only for 75 tests, may be used over a much broader range of possible foreign entities. In such embodiments, a positive reaction (described below) for any group of foreign entities would require a subsequent test or tests to determine which of the multitude of foreign entities contained in that test sample was the specific foreign entity which caused the positive reaction. It is also preferred that an amount of the suspension medium, equal to that introduced to the test samples, be added to the control sample. This allows the direct comparison of the two samples, since they will have generally identical counts of leukocytes (before the studied reaction), and generally similar volumes.

At this point, each sample, i.e. the control sample and each test sample, will contain a mixture of suspension medium and whole blood, which in turn includes leukocytes, red blood cells and platelets. In addition, the test samples will have the solutions of foreign entities therein. Since all three types of blood cells are of importance to this test, it is important that each be measured separately.

It is preferred that counter 12 is Coulter Counter Model Z-M, which may be set to count the number of particles within a given size range. Thus, since platelets are much smaller than red and white blood cells, it is simple to avoid counting them by setting the minimum particle size at a size greater than that of platelets, and less than that of the red and white blood cells, for example 3.5 microns. Similarly, it is simple to count only those cells by setting the maximum level of counter 12 to a similar level.

No such simple method of counting red and white blood cells is presently available, however. It is possible to separate these cells in known mechanical methods, although this method is time consuming.

A preferred method utilizes the fact that red blood cells exist in human serum in a ratio of 1000:1 to leukocytes. Thus, it is preferred that the red blood cells be counted first. This may be taken as a straight count, since the relatively small number of white blood cells existing in blood serum may be disregarded when the count is made. The small error which may occur (on the order of 0.1 per cent) may be disregarded, since that error is generally far smaller than the error factor of the equipment, and is also far smaller than the amount of change expected in the case of a positive reaction. After the red blood cells are counted, then it is desired to count the leukocytes.

To avoid counting red blood cells, which, as stated, are of roughly comparable size to the leukocytes, it is preferred that those cells be eliminated. This is preferably accomplished by adding a substance which will immediately cause red blood cells to disintegrate (a "lysing" substance), for example a solution comprising one percent Saponin (Coulter) and the remainder bacteriostatic water. Alternatively, the red blood cells may be mechanically removed from the sample. This solution may also serve to lyse some reacted leukocytes in the test sample, thereby enhancing the observable effects of the reaction (if any) between the leukocytes and the foreign entity (together with the antibodies). After the red blood cells and a portion of the reacted leukocytes are eliminated, preferably after a period of about 30 seconds after introduction of the lysing agent, and counter 12 is set to the predetermined minimum size level, counter 12 may be used to count and size the leukocytes of the control sample. The results of the counting and sizing of the control sample are used as a reference for comparison of the results of the same counts performed on the control samples. It will be appreciated that the platelets may be counted either before or after the counting of the red blood cells but before the leukocytes. Since platelets are not directly affected by the lysing substances, the order of counting the platelets and red blood cells is a mere matter of choice. However, the red blood cells, when lysed, may create particles which are of a size on the order of platelets, so the platelets should be counted prior to lysing the red blood cells, which means prior to counting the leukocytes. Of course, if some other manner of separating the three types of blood cells is used, such as mechanical separation, then the order of counting is even more a matter of mere choice.

If the blood cells in one or more of the test samples react to the presence of any foreign entity, then they will react in the manner described, i.e. by getting larger, then breaking up and finally dissolving. These reactions will cause a distortion of the size distributions of the blood cells in the positive test samples, since the number of blood cells in lower volumetric size ranges will diminish, and the number of blood cells in higher volumetric size ranges will increase, thereby producing a shift in the size-distributions of the counted blood cells.

The output of counter 12 may be read visually, to determine if the number of blood cells in the test sample is less than that of the control (indicating positive reaction) or it may be input to analyzer 14, such as the Coulter Channelyzer, to obtain cellular population distributions of the number of red blood cells present in each of a plurality of size-distribution ranges. This is referred to as "sizing".

The output of analyzer 14 may also be input to computer 18 through interface 16, in known fashion, to store the data and compare automatically the results of the count of each test sample to that of the control sample as well as the size-distribution of the blood cells. If the number of blood cells in a test sample is less than that of the control by more than the error factor of counter 12, then there is a positive reaction. The error factor of each piece of equipment used is readily available from its manufacturer, and may vary from manufacturer to manufacturer. In addition, if the comparison of the size-distribution results indicate enlargement of blood cells, then there is also a positive reaction. Once all comparisons are made, the output of computer 18 may be displayed by any means desired, such as printer 22 or video display 24, and may also be stored on disc 20 for future reference.

Storing data is useful for later reference on the same subject at a later date. If a control sample of a subject's blood taken at a subsequent time is vastly different from that of a first test, then there may be an indication of a vast change in the state of that subject's immune system. This would suggest further testing to determine the cause of such a change.

It is here noted that the above description was made based on the assumption that a foreign entity is added to the test samples to produce thereby a reaction. This may not be effective in all instances. Certain non-immunologic reactions cause cellular reactions similar to the above-described immunologic reaction. In such cases, the inventive test is still effective in diagnosing the malady, so long as the non-immunolgic reaction causes changes in cellular size, or cellular destruction.

As described thus far, the inventive test requires the presence of two elements in the blood: antibodies and blood cells, so that the introduction of the third component of the studied reaction, the foreign entity, will cause the measurd reactions to occur. Not all individuals produce antibodies appropriate for all foreign entities, however. This is particularly true for carcinogens, for which no naturally occurring antibodies are present in the blood of most people.

The test will still work, however, with one modification. Instead of adding the foreign entity to the control sample, a different substance, such as an antibody, is introduced thereto. This modification will enable the diagnosis of a malady caused by the ingestion of a foreign entity by the subject, even if the subject does not produce the antibodies on his own. The addition of the antibodies to the test sample completes the requirement that all three components of the reaction: antibodies, foreign entity and blood cells, be present in the test sample.

For example, with respect to the testing for a specific cancer, if the carcinogenic foreign entity is present in the subject's blood, the introduction of a monoclonal antibody specific to that carcinogenic foreign entity will enable the blood cells in the subject's blood to attack the carcinogen, and the normally observed reaction will take place.

So long as an antibody is known for any specific foreign entity, the malady caused by the ingestion of that foreign entity may be diagnosed.

In this fashion, it may be objectively determined if the subject has a reaction to any of the tested foreign entities, and is therefore suffering from any of the tested maladies. Such a test may be used to test for any malady affecting the immune system, from influenza, to AIDS, to cancer. Any such malady may be tested for, and at a stage where treatment will be most effective, i.e. at as early a stage as possible.

The discovery that red blood cells react to the presence of certain entities may also lead to a course of treatment for certain anemias, wherein those foreign entities which cause reactions in the subject's red blood cells are removed from the subject's environment, thereby removing a possible cause of depletion of the subject's red blood cells.

It is also here noted that there is nothing in this description which will limit the application of this method to the human immune system. It is believed that this method is equally applicable, for example, in the field of veterinary medicine for any animal having an immune system.

The entire apparatus may be automated so that once the sample of blood is drawn from the subject there need be no further human intervention or action until the results are complete.

## Claims

1. A method for diagnosing the presence or absence of a malady affecting the immune system of a subject which is indicated by the presence of a foreign entity, said method comprising the steps of:
counting a number of leukocytes in a first blood sample drawn from said subject:
mixing an antibody specific to said malady with a second blood sample drawn from said subject to form a mixture, and thereby allow leukocytes of said second blood sample to react with said antibody and said foreign entity if present;
counting a number of unreacted and reacted leukocytes in said mixture; and
comparing said number of said leukocytes counted in said first blood sample with said number of unreacted and reacted leukocytes counted in said mixture, to determine if said leukocytes in said second blood sample reacted with said antibody, thereby diagnosing the presence or absence of said malady in said subject.

2. The method of claim 1, wherein said counting a number of leukocytes of at least one of said first blood sample and said mixture is performed by counting a number of leukocytes within each of a plurality of varying size-distribution ranges.

3. The method of claim 2, further comprising the step of:
producing a graph showing said number of leukocytes counted in each of said plurality of size-distribution ranges.

4. The method of claim 1, further comprising the step of:
lysing a portion of any red blood cells present in said mixture prior to counting said number of unreacted and reacted leukocytes therein.

5. The method of claim 1, further comprising the step of:
lysing a portion of said reacted leukocytes in said mixture prior to counting said number of unreacted and reacted leukocytes therein.

6. The method of claim 1, further comprising the step of:
lysing a portion of any red blood cells present in said first blood sample prior to counting said number of leukocytes therein.

7. The method of claim 1, further comprising the steps of:
separating a sample of said blood of said subject into said first blood sample and said second blood sample, each of said first blood sample and said second blood sample having approximately equal distributions of leukocytes therein;
lysing a portion of any red blood cells in said first blood sample;
counting a number of leukocytes in said first blood sample; and
lysing a portion of any red blood cells in said mixture before performing the steps of counting the number of unreacted and reacted leukocytes in said mixture and comparing said number of leukocytes in said mixture with said number of leukocytes in said first blood sample, to determine if at least a portion of said leukocytes in said second blood sample reacted with said antibody, thereby indicating the presence or absence of said malady in said subject.

8. The method of claim 7, wherein at least one of said counting of said number of leukocytes in said first blood sample and said counting of the number of leukocytes in said mixture are performed by counting a number of leukocytes within each of a plurality of varying size-distribution ranges therein, and further comprising the steps of
generating a size-distribution graph showing a size-distribution of leukocytes in each of said first blood sample and said mixture; and
comparing said size-distribution graph showing said size-distribution of said leukocytes in said first blood sample with said size-distribution graph showing said size-distribution of leukocytes in said mixture;
whereby the presence of said malady in said subject is indicated by a significant difference therebetween.

9. The method of claim 7, wherein the determination of whether said antibody has caused a reaction with said second blood sample is positive when said number of unreacted leukocytes counted in said mixture is less than said number of leukocytes counted in said first blood sample by an amount greater than an error factor of the equipment used.

10. A method for diagnosing the presence or absence of a malady affecting the immune system of a subject which is indicated by the presence of a foreign entity, said method comprising the steps of:
sizing a number of leukocytes in a first blood sample drawn from said subject;
mixing an antibody specific to said malady with a second blood sample drawn from said subject to form a mixture, and thereby allow leukocytes present in said second blood sample to react with said antibody and said foreign entity if present;
sizing a number of unreacted and reacted leukocytes in said mixture; and
comparing said sized leukocytes of said first blood sample with said sized unreacted and reacted leukocytes of said mixture, to determine thereby if at least a portion of said leukocytes in said second blood sample reacted with said antibody, whereby the presence of said malady in said subject is indicated by a significant difference therebetween.

11. The method of claim 10, further comprising the step of:
lysing a portion of any red blood cells present in said first blood sample prior to sizing said leukocytes therein.

12. The method of claim 10, further comprising the step of:
lysing a portion of said reacted leukocytes in said mixture prior to sizing said unreacted and reacted leukocytes therein.

13. The method of claim 10, wherein at least one of said sizing of said leukocytes in said first blood sample and said sizing of said unreacted leukocytes in said mixture is performed by counting a number of leukocytes within each of a plurality of varying size-distribution ranges.

14. The method of claim 10, further comprising the steps of:
separating a blood sample drawn from said subject into said first blood sample and said second blood sample, each of said first blood sample and said second blood sample having approximately equal distributions of leukocytes therein;
lysing a portion of red blood cells in said first blood sample;
sizing said leukocytes in said first blood sample thereby generating a size distribution of said leukocytes in said first blood sample;
lysing a portion of any red blood cells in said mixture;
lysing a portion of any reacted leukocytes in said mixture;
sizing said leukocytes in said mixture thereby generating a size distribution of said leukocytes in said mixture; and
comparing said size distribution of said leukocytes in said mixture with said size distribution of said leukocytes in said first blood sample, and determine thereby if said malady is present in said subject.

15. The method of claim 14, wherein at least one of said sizing of said leukocytes in said first blood sample and said sizing of said leukocytes in said mixture is performed by counting a number of leukocytes within each of a plurality of varying size-distribution ranges, and
generating a size-distribution of the counts of each of said first blood sample and said mixture; and
comparing said size-distribution of said leukocytes in said first blood sample with said size-distribution of said leukocytes in said mixture, to determine thereby if said malady is present in said subject.

16. The method of claim 14, wherein the determination of whether said malady is present in said subject is positive when said size-distribution of said leukocytes in said mixture differs from said size-distribution of said leukocytes in said first blood sample to a degree greater than an error factor of the equipment used.

17. A method for diagnosing the presence or absence of a malady affecting the immune system of a subject, said method comprising the steps of:
counting a number of blood cells of at least one of a first and second type selected from the group consisting of red blood cells and platelets, in a first blood sample drawn from said subject;
mixing a specific foreign entity known to provoke said malady or an antibody specific to said malady with a second blood sample drawn from said subject to form a mixture, and thereby allow blood cells of said second blood sample to react with said foreign entity or said antibody;
counting a number of unreacted and reacted blood cells of said at least one of said first and second types, respectively in said mixture; and
comparing the number of said at least one of said first and second type of blood cells counted in said first blood sample with said number of unreacted and reacted blood cells of said at least one of said first and second type, respectively, counted in said mixture, to determine if said blood cells in said second blood sample reacted with said foreign entity or said antibody, thereby producing at least one result for diagnosing the presence or absence of said malady in said subject.

18. The method of claim 17, wherein said counting a number of at least one of said first and second type of blood cells of at least one of said first blood sample and said mixture is performed by counting a number of blood cells within each of a plurality of varying size-distribution ranges.

19. The method of claim 18, further comprising the step of:
producing a graph showing said number of blood cells counted in each of said plurality of size-distribution ranges.

20. The method according to any one of claims 17 to 19, wherein said first and second blood samples further include leukocytes and each of said first blood sample and said second blood sample have approximately equal distributions of blood cells therein, said method further comprising the steps of:
lysing at least a portion of said blood cells in said first blood sample;
counting a number of leucocytes in said first blood sample;
mixing a specific foreign entity known to provoke said malady or an antibody specific to said malady with said second blood sample, thereby producing a mixture;
lysing a portion of said blood cells in said mixture;
counting a number of leucocytes in said mixture; and
comparing said number of leucocytes in said mixture with said number of said leucocytes in said first blood sample, thereby producing a further result, whereby the presence or absence of said malady may also be determined by said further result.

21. The method of claim 20, wherein at least one of said numbers of leucocytes counted is stored in a memory.

22. The method of claim 21, further comprising the steps of:
collecting a second sample of blood from said subject;
counting a number of leucocytes of said second sample; and
comparing said number of leucocytes with said number of leucocytes stored in said memory, whereby any change in a number or distribution of leucocytes in said blood of said subject over time may be discovered.

## Patentansprüche

1. Ein Verfahren zum Diagnostizieren der Anwesenheit oder Abwesenheit einer Krankheit, die das Immunsystem eines Subjekts beeinträchtigt, was durch die Anwesenheit einer fremden Einheit angezeigt wird, wobei das Verfahren folgende Schritte umfaßt:
Zählen einer Anzahl Leukozyten in einer ersten Blutprobe, die dem Subjekt entnommen wurde:
Mischen eines für die Krankheit spezifischen Antikörpers mit einer zweiten Blutprobe, die dem Subjekt entnommen wurde, um eine Mischung zu bilden und es dadurch den Leukozyten der zweiten Blutprobe zu ermöglichen, mit dem Antikörper und der fremden Einheit - so vorhanden - zu reagieren;
Zählen einer Anzahl nicht reagierter und reagierter Leukozyten in der Mischung; und
Vergleichen der Anzahl der in der ersten Blutprobe gezählten Leukozyten mit der Anzahl der in der Mischung gezählten nicht reagierten und reagierten Leukozyten, um zu bestimmen, ob die Leukozyten in der zweiten Blutprobe mit dem Antikörper reagiert haben, und dadurch die Anwesenheit oder Abwesenheit der Krankheit in dem Subjekt zu diagnostizieren.

2. Das Verfahren nach Anspruch 1, wobei das Zählen einer Anzahl Leukozyten wenigstens einer der ersten Blutprobe und der Mischung durchgeführt wird durch Zählen einer Anzahl Leukozyten innerhalb jedes einer Mehrzahl variierender Größenverteilungsbereiche.

3. Das Verfahren nach Anspruch 2, weiterhin umfassend den Schritt:
Erstellen eines Graphen, der die Anzahl der in jedem der Mehrzahl der Größenverteilungsbereichen gezählten Leukozyten zeigt.

4. Das Verfahren nach Anspruch 1, weiterhin umfassend den Schritt:
Lysinieren eines Teils jeglicher in der Mischung vorhandener roter Blutkörperchen vor dem Zählen der Anzahl darin enthaltener nicht reagierter und reagierter Leukozyten.

5. Das Verfahren nach Anspruch 1, weiterhin umfassend den Schritt:
Lysinieren eines Teils der reagierten Leukozyten in der Mischung vor dem Zählen der Anzahl darin enthaltener nicht reagierter und reagierter Leukozyten.

6. Das Verfahren nach Anspruch 1, weiterhin umfassend den Schritt:
Lysinieren eines Teils jeglicher in der Mischung vorhandener roter Blutkörperchen vor dem Zählen der Anzahl darin enthaltener Leukozyten.

7. Das Verfahren nach Anspruch 1, weiterhin umfassend den Schritt:
Trennen einer Blutprobe des Subjekts in die erste Blutprobe und die zweite Blutprobe, wobei die erste und die zweite Blutprobe darin ungefähr gleiche Leukozytenverteilungen aufweisen;
Lysinieren eines Teils jeglicher roter Blutkörperchen in der ersten Blutprobe;
Zählen einer Anzahl Leukozyten in der ersten Blutprobe; und
Lysinieren eines Teils jeglicher roter Blutkörperchen in der Mischung vor dem Ausführen der Schritte des Zählens der Anzahl nicht reagierter und reagierter Leukozyten in der Mischung und des Vergleichens der Anzahl Leukozyten in der Mischung mit der Anzahl Leukozyten in der ersten Blutprobe, um zu bestimmen, ob wenigstens ein Teil der Leukozyten in der zweiten Blutprobe mit dem Antikörper reagiert hat, um dadurch die Anwesenheit oder Abwesenheit der Krankheit in dem Subjekt anzuzeigen.

8. Das Verfahren nach Anspruch 7, wobei wenistens eine Maßnahme des Zählens der Anzahl Leukozyten in der ersten Blutprobe und des Zählens der Anzahl Leukozyten in der Mischung durch Zählen einer Anzahl Leukozyten innerhalb jeder der Mehrzahl variierender Größenverteilungsbereiche darin durchgeführt wird, und weiterhin umfassend die Schritte
des Erzeugens eines Größenverteilungsgraphen, der eine Größenverteilung von Leukozyten in jeweils der ersten Blutprobe und der Mischung zeigt; und
des Vergleichens des Größenverteilungsgraphen, der die Größenverteilung der Leukozyten in der ersten Blutprobe zeigt, mit dem Größenverteilungsgraphen, der die Größenverteilung von Leukozyten in der Mischung zeigt;
wodurch die Anwesenheit der Krankheit in dem Subjekt durch einen signifikanten Unterschied zwischen den beiden angezeigt wird.

9. Das Verfahren nach Anspruch 7, wobei die Bestimmung, ob der Antikörper eine Reaktion mit der zweiten Blutprobe ausgelöst hat, positiv ist, wenn die Anzahl in der Mischung gezählter nicht reagierter Leukozyten um einen Betrag, größer als der Fehlerfaktor der verwendeten Ausrüstung, kleiner ist als die Anzahl Leukozyten, die in der ersten Blutprobe gezählt ist.

10. Ein Verfahren zum Diagnostizieren der Anwesenheit oder Abwesenheit einer Krankheit, die das Immunsystem eines Subjekts beeinträchtigt, was durch die Anwesenheit einer fremden Einheit angezeigt wird, wobei das Verfahren folgende Schritte umfaßt:
(Größen-) Klassieren einer Anzahl Leukozyten in einer ersten Blutprobe, die dem Subjekt entnommen wurde:
Mischen eines für die Krankheit spezifischen Antikörpers mit einer zweiten Blutprobe, die dem Subjekt entnommen wurde, um eine Mischung zu bilden und es dadurch den Leukozyten der zweiten Blutprobe zu ermöglichen, mit dem Antikörper und der fremden Einheit - so vorhanden - zu reagieren;
(Größen-) Klassieren einer Anzahl nicht reagierter und reagierter Leukozyten in der Mischung; und
Vergleichen der klassierten Leukozyten der ersten Blutprobe mit den klassierten nicht reagierten und reagierten Leukozyten der Mischung, um dadurch zu bestimmen, ob wenigstens ein Teil der Leukozyten in der zweiten Blutprobe mit dem Antikörper reagiert hat, wodurch die Anwesenheit der Krankheit in dem Subjekt durch einen signifikanten Unterschied dazwischen angezeigt wird.

11. Das Verfahren nach Anspruch 10, weiterhin umfassend den Schritt:
Lysinieren eines Teils jeglicher in der Mischung vorhandener roter Blutkörperchen vor dem Klassieren der darin enthaltenen Leukozyten.

12. Das Verfahren nach Anspruch 10, weiterhin umfassend den Schritt:
Lysinieren eines Teils der reagierten Leukozyten in der Mischung vor dem Klassieren der darin enthaltenen nicht reagierten und reagierten Leukozyten.

13. Das Verfahren nach Anspruch 10, wobei wenigstens eine Maßnahme des Klassierens der Leukozyten in der ersten Blutprobe und des Klassierens der nicht reagierten Leukozyten in der Mischung durch das Zählen einer Anzahl Leukozyten jeweils in einer Mehrzahl variierender Größenverteilungsbereiche durchgeführt wird.

14. Das Verfahren nach Anspruch 10, weiterhin umfassend die Schritte:
Trennen einer von dem Subjekt gezogenen Blutprobe in die erste Blutprobe und die zweite Blutprobe, wobei jeweils die erste und die zweite Blutprobe darin ungefähr gleiche Leukozytenverteilungen aufweisen;
Lysinieren eines Teils der roten Blutkörperchen in der ersten Blutprobe;
Klassieren der Leukozyten in der ersten Blutprobe, um dadurch eine Größenverteilung der Leukozyten in der ersten Blutprobe zu erzeugen;
Lysinieren eines Teils jeglicher roter Blutkörperchen in der Mischung;
Lysinieren eines Teils jeglicher reagierter Leukozyten in der Mischung;
Klassieren der Leukozyten in der Mischung, um dadurch eine Größenverteilung der Leukozyten in der Mischung zu erzeugen; und
Vergleichen der Größenverteilung der Leukozyten in der Mischung mit der Größenverteilung der Leukozyten in der ersten Blutprobe, und dadurch Bestimmen, ob die Krankheit in dem Subjekt vorhanden ist.

15. Das Verfahren nach Anspruch 14, wobei wenigstens eine der Maßnahmen des Klassierens der Leukozyten in der ersten Blutprobe und des Klassierens der Leukozyten in der Mischung durch Zählen einer Anzahl Leukozyten in jeweils einer Mehrzahl variierender Größenverteilungsbereiche durchgeführt wird, und
Erzeugen einer Größenverteilung der Zählungen jeweils der ersten Blutprobe und der Mischung; und
Vergleichen der Größenverteilung der Leukozyten in der ersten Blutprobe mit der Größenverteilung der Leukozyten in der Mischung, um dadurch zu bestimmen, ob die Krankheit in dem Subjekt vorhanden ist.

16. Das Verfahren nach Anspruch 14, wobei die Bestimmung, ob die Krankheit in dem Subjekt vorliegt, positiv ist, wenn die Größenveteilung der Leukozyten in der Mischung von der Größenverteilung der Leukozyten in der ersten Blutprobe um einen Grad abweicht, der größer als der Fehlerfaktor der verwendeten Ausrüstung ist.

17. Ein Verfahren zum Diagnostizieren der Anwesenheit oder Abwesenheit einer Krankheit, die das Immunsystem eines Subjekts beeinträchtigt, wobei das Verfahren folgende Schritte umfaßt:
Zählen einer Anzahl Blutkörperchen von wenigstens einem eines ersten und zweiten Typs, ausgewählt aus der aus roten Blutkörperchen und Plättchen bestehenden Gruppe, in einer ersten Blutprobe, die dem Subjekt entnommen wurde;
Mischen einer spezifischen fremden Einheit, von der bekannt ist, daß sie die Krankheit provoziert, oder eines für die Krankheit spezifischen Antikörpers mit einer zweiten Blutprobe, die dem Subjekt entnommen wurde, um eine Mischung zu bilden und es dadurch den Blutzellen der zweiten Blutprobe zu ermöglichen, mit der fremden Einheit oder dem Antikörper zu reagieren;
Zählen einer Anzahl nicht reagierter und reagierter Blutkörperchen wenigstens eines des ersten und des zweiten Typs in der Mischung; und
Vergleichen der Anzahl der in der ersten Blutprobe gezählten Blutkörperchen des wenigstens ersten und zweiten Typs mit der Anzahl der in der Mischung gezählten nicht reagierten und reagierten Blutkörperchen des jeweils wenigstens ersten und zweiten Typs, um zu bestimmen, ob die Blutkörperchen in der zweiten Blutprobe mit der fremden Einheit oder dem Antikörper reagiert haben, um dadurch weingstens ein Ergebnis zur Diagnose der Anwesenheit oder Abwesenheit der Krankheit in dem Subjekt zu liefern.

18. Das Verfahren nach Anspruch 17, wobei das Zählen einer Anzahl wenigstens eines des ersten und zweiten Typs Blutkörperchen wenigstens einer der ersten Blutprobe und der Mischung durch das Zählen einer Anzahl Blutkörperchen in jeweils einer Mehrzahl variierender Größenverteilungsbereiche durchgeführt wird.

19. Das Verfahren nach Anspruch 18, weiterhin umfassend den Schritt:
Liefern eines Graphs, der die Anzahl Blutkörperchen zeigt, die in jeder der Mehrzahl von Größenverteilungsbereichen gezählt worden ist.

20. Das Verfahren nach einem der Ansprüche 17 bis 19, wobei die erste und zweite Blutprobe des weiteren Leukozyten umfassen und jede der ersten und der zweiten Blutprobe in sich ungefähr gleiche Verteilungen von Blutzellen aufweisen und das Verfahren weiterhin die Schritte umfaßt:
Lysinieren wenigstens eines Teils der Blutkörperchen in der ersten Blutprobe;
Zählen einer Anzahl Leukozyten in der ersten Blutprobe;
Mischen einer spezifischen fremden Einheit, von der bekannt ist, daß sie die Krankheit provoziert, oder eines für die Krankheit spezifischen Antikörpers mit der zweiten Blutprobe, und dabei Herstellen einer Mischung;
Lysinieren eines Teils der Blutkörperchen in der Mischung;
Zählen einer Anzahl Leukozyten in der Mischung; und
Vergleichen der Anzahl Leukozyten in der Mischung mit der Anzahl Leukozyten in der ersten Blutprobe, dabei Liefern eines weiteren Ergebnisses, wodurch die Anwesenheit oder Abwesenheit der Krankheit ebenfalls durch das weitere Ergebnis bestimmt werden kann.

21. Das Verfahren nach Anspruch 20, wobei wenigstens eine der gezählten Leukozytenanzahlen in einem Speicher gespeichert wird.

22. Das Verfahren nach Anspruch 21, weiterhin umfassend die Schritte:
Nehmen einer zweiten Blutprobe von dem Subjekt;
Zählen einer Anzahl Leukozyten der zweiten Probe; und
Vergleichen der Anzahl Leukozyten mit der Anzahl Leukozyten, die in dem Speicher gespeichert ist, wodurch jegliche Veränderung in der Anzahl oder Verteilung von Leukozyten in dem Blut des Subjekts mit der Zeit offenbart werden kann.

## Revendications

1. Procédé de diagnostic de la présence ou de l'absence d'une maladie affectant le système immun d'un patient laquelle est indiquée par la présence d'une entité étrangère, ledit procédé comprenant les étapes consistant à :
compter le nombre de leucocytes dans un premier échantillon de sang prélevé sur le patient ;
mélanger un anticorps spécifique à ladite maladie avec un second échantillon de sang prélevé sur ledit patient pour former un mélange, et permettre ainsi aux leucocytes dudit second échantillon de sang de réagir avec ledit anticorps et ladite entité étrangère si elle est présente ;
compter le nombre de leucocytes ayant réagi et n'ayant pas réagi dans ledit mélange ; et
comparer ledit nombre desdits leucocytes comptés dans ledit premier échantillon de sang avec ledit nombre de leucocytes n'ayant pas réagi et ayant réagi comptés dans ledit mélange, de façon à déterminer si lesdits leucocytes dans ledit second échantillon de sang ont réagi avec ledit anticorps, de façon à diagnostiquer la présence ou l'absence de ladite maladie chez ledit patient.

2. Procédé selon la revendication 1, dans lequel ledit comptage du nombre des leucocytes d'au moins ledit premier échantillon de sang et dudit mélange est effectué en comptant le nombre de leucocytes à l'intérieur de chacune d'une pluralité de plages de distribution de dimensions variables.

3. Procédé selon la revendication 2, comprenant en outre l'étape consistant à :
produire un graphique montrant ledit nombre de leucocytes comptés dans chacune de ladite pluralité de plages de distribution de dimensions.

4. Procédé selon la revendication 1 comprenant en outre l'étape consistant à :
lyser une partie de chacun des globules rouges présents dans ledit mélange avant de compter ledit nombre de leucocytes n'ayant pas réagi et ayant réagi qui y sont contenus.

5. Procédé selon la revendication 1, comprenant en outre l'étape consistant à :
lyser une partie desdits leucocytes ayant réagi dans ledit mélange avant de compter ledit nombre de leucocytes n'ayant pas réagi et ayant réagi qui y sont contenus.

6. Procédé selon la revendication 1, comprenant en outre l'étape consistant à :
lyser une partie de chacun des globules rouges présents dans ledit premier échantillon de sang avant de compter ledit nombre de leucocytes qui y sont contenus.

7. Procédé selon la revendication 1, comprenant en outre les étapes consistant à :
séparer un échantillon dudit sang provenant dudit patient de façon à former ledit premier échantillon de sang et ledit second échantillon de sang, chacun d'entre eux ayant approximativement des distributions égales de leucocytes qui y sont contenus ;
lyser une partie de chacun des globules rouges dans ledit premier échantillon de sang ;
compter le nombre de leucocytes contenus dans ledit premier échantillon de sang ; et
lyser une partie de chacun des globules rouges dans ledit mélange avant d'effectuer les étapes de comptage du nombre de leucocytes n'ayant pas réagi et ayant réagi dans ledit mélange et comparer ledit nombre de leucocytes dans ledit mélange avec ledit nombre de leucocytes dans ledit premier échantillon de sang, de façon à déterminer si au moins une partie desdits leucocytes dans le second échantillon de sang ont réagi avec ledit anticorps, indiquant de la sorte la présence ou l'absence de ladite maladie chez ledit patient.

8. Procédé selon la revendication 7, dans lequel au moins l'une desdites opérations de comptage dudit nombre de leucocytes dans ledit premier échantillon de sang et de comptage du nombre de leucocytes dans ledit mélange est effectuée en comptant un nombre de leucocytes à l'intérieur de chacune d'une pluralité de plages de distribution de dimensions variables, et comprenant en outre les étapes consistant à :
engendrer un graphique de distribution de dimensions montrant la distribution de dimensions des leucocytes dans chacun dudit premier échantillon de sang et dudit mélange ; et
comparer ledit graphique de distribution de dimensions montrant ladite distribution de dimensions desdits leucocytes dans ledit premier échantillon de sang, ledit graphique de distribution de dimensions montrant ladite distribution de dimensions des leucocytes dans ledit mélange ;
grâce à quoi, la présence de ladite maladie chez ledit patient est indiquée par une différence significative entre ces graphiques.

9. Procédé selon la revendication 7, dans lequel on détermine de façon positive si ledit anticorps a provoqué une réaction avec ledit second échantillon de sang lorsque ledit nombre de leucocytes n'ayant pas réagi comptés dans ledit mélange est inférieur audit nombre de leucocytes comptés dans ledit premier échantillon de sang d'une quantité supérieure à un facteur d'erreur de l'appareillage utilisé.

10. Procédé de diagnostic de la présence ou de l'absence d'une maladie affectant le système immun d'un patient qui est indiquée par la présence d'une entité étrangère, ledit procédé comprenant les étapes consistant à :
calibrer un nombre de leucocytes dans un premier échantillon de sang prélevé à un patient ;
mélanger un anticorps spécifique à ladite maladie avec un second échantillon de sang prélevé audit patient de façon à former un mélange, et permettre ainsi aux leucocytes présents dans ledit second échantillon de sang de réagir avec ledit anticorps et ladite entité étrangère si elle est présente ;
calibrer un nombre de leucocytes n'ayant pas réagi et ayant réagi dans ledit mélange ; et
comparer lesdits leucocytes calibrés dudit premier échantillon de sang avec lesdits leucocytes calibrés n'ayant pas réagi et ayant réagi dudit mélange, de façon à déterminer ainsi si au moins une partie desdits leucocytes contenus dans le second échantillon de sang ont réagi avec ledit anticorps, indiquant ainsi la présence de ladite maladie chez ledit patient sous forme d'une différence significative résultant de cette comparaison.

11. Procédé selon la revendication 10, comprenant en outre l'étape consistant à :
lyser une partie de chacun des globules rouges présents dans ledit premier échantillon de sang avant de calibrer lesdits leucocytes qui y sont contenus.

12. Procédé selon la revendication 10, comprenant en outre l'étape consistant à :
lyser une partie desdits leucocytes ayant réagi dans ledit mélange avant de calibrer lesdits leucocytes n'ayant pas réagi et ayant réagi qui y sont contenus.

13. Procédé selon la revendication 10, dans lequel au moins l'un des calibrages desdits leucocytes dans ledit premier échantillon de sang et ledit calibrage desdits leucocytes n'ayant pas réagi dans ledit mélange sont effectués en comptant le nombre de leucocytes contenus dans chacune d'une pluralité de plages de distribution de dimensions variables.

14. Procédé selon la revendication 10, comprenant en outre les étapes consistant à :
séparer un échantillon de sang prélevé à un patient en ledit premier échantillon de sang et ledit second échantillon de sang, chacun desdits premier échantillon de sang et second échantillon de sang contenant approximativement des distributions égales de leucocytes ;
lyser une partie des globules rouges dans ledit premier échantillon de sang ;
calibrer lesdits leucocytes dans ledit premier échantillon de sang de façon à engendrer une distribution de dimensions desdits leucocytes dans ledit premier échantillon de sang ;
lyser une partie de chacun des globules rouges contenus dans ledit mélange ;
lyser une partie de chacun des leucocytes ayant réagi dans ledit mélange ;
calibrer lesdits leucocytes dans ledit mélange de façon à engendrer une distribution de dimensions desdits leucocytes dans ledit mélange ; et
comparer ladite distribution de dimensions desdits leucocytes dans ledit mélange avec ladite distribution de dimensions desdits leucocytes dans ledit premier échantillon de sang, et déterminer par suite si ladite maladie est présente chez ledit patient.

15. Procédé selon la revendication 14, dans lequel au moins l'un desdits calibrages desdits leucocytes dans ledit premier échantillon de sang et ledit calibrage desdits leucocytes dans ledit mélange sont effectués en comptant le nombre de leucocytes contenus dans chacune d'une pluralité de plages de distribution de dimensions variables, et dans lequel :
on engendre une distribution de dimensions des comptes de chacun dudit premier échantillon de sang et dudit mélange ; et
on compare ladite distribution de dimensions desdits leucocytes contenus dans ledit premier échantillon de sang avec ladite distribution de dimensions desdits leucocytes dans ledit mélange, de façon à déterminer par cette comparaison si la maladie est présente chez ledit patient.

16. Procédé selon la revendication 14, dans lequel on détermine de façon positive si la maladie est présente chez ledit patient lorsque ladite distribution de dimensions desdits leucocytes dans le mélange diffère de ladite distribution de dimensions desdits leucocytes dans ledit premier échantillon de sang d'un degré supérieur à un facteur d'erreur de l'appareillage utilisé.

17. Procédé de diagnostic de la présence ou de l'absence d'une maladie affectant le système immun d'un patient, ledit procédé comprenant les étapes consistant à :
compter le nombre des cellules sanguines d'au moins un premier et un second types choisis parmi le groupe comprenant les globules rouges et les plaquettes, dans un premier échantillon de sang prélevé audit patient ;
mélanger une entité étrangère spécifique connue pour provoquer ladite maladie ou un anticorps spécifique à ladite maladie avec un second échantillon de sang prélevé audit patient de façon à former un mélange et permettre ainsi aux cellules sanguines dudit second échantillon de sang de réagir avec ladite entité étrangère ou ledit anticorps;
compter le nombre de cellules sanguines n'ayant pas réagi et ayant réagi de l'un au moins dudit premier et dudit second types, respectivement, dans ledit mélange ; et
comparer le nombre d'au moins l'une desdites cellules de sang dudit premier et second types comptées dans ledit premier échantillon de sang avec ledit nombre de cellules sanguines n'ayant pas réagi et ayant réagi de l'un au moins dudit premier et second types, respectivement, comptées dans ledit mélange, de façon à déterminer si lesdites cellules sanguines dans ledit second échantillon de sang ont réagi avec ladite entité étrangère ou ledit anticorps, de façon à produire au moins un résultat pour diagnostiquer la présence ou l'absence de ladite maladie chez ledit patient.

18. Procédé selon la revendication 17, dans lequel ledit comptage du nombre des cellules sanguines d'au moins l'un desdits premier et second types d'au moins l'un dudit premier échantillon de sang et dudit mélange est effectué en comptant le nombre de cellules sanguines à l'intérieur de chacune d'une pluralité de plages de distribution de dimensions variables.

19. Procédé selon la revendication 18, comprenant en outre l'étape consistant à :
produire un graphique montrant ledit nombre de cellules sanguines comptées dans chacune de ladite pluralité de plages de distribution de dimensions.

20. Procédé selon l'une quelconque des revendications 17 à 19, dans lequel ledit premier et ledit second échantillons de sang comprennent en outre des leucocytes et chacun dudit premier échantillon de sang et dudit second échantillon de sang contiennent approximativement des distributions égales de cellules sanguines, ledit procédé comprenant en outre les étapes consistant à :
lyser au moins une partie desdites cellules sanguines dans ledit premier échantillon de sang ;
compter le nombre de leucocytes contenus dans ledit premier échantillon de sang ;
mélanger une entité étrangère spécifique connue pour provoquer ladite maladie ou un anticorps spécifique à ladite maladie avec ledit échantillon de sang, de façon à produire un mélange ;
lyser une partie desdites cellules sanguines dans ledit mélange ;
compter le nombre de leucocytes dans ledit mélange ; et
comparer ledit nombre de leucocytes dans ledit mélange avec ledit nombre desdits leucocytes dans ledit premier échantillon de sang, ceci permettant de produire un nouveau résultat, grâce auquel la présence ou l'absence de ladite maladie peut également être déterminée par ledit nouveau résultat.

21. Procédé selon la revendication 20, dans lequel au moins l'un desdits nombres des leucocytes comptés est stocké dans une mémoire.

22. Procédé selon la revendication 21, comprenant en outre les étapes consistant à :
recueillir un second échantillon de sang provenant dudit patient ;
compter le nombre de leucocytes dans ledit second échantillon ; et
comparer ledit nombre de leucocytes avec ledit nombre de leucocytes stocké dans ladite mémoire, grâce à quoi cette modification dans le nombre ou la distribution des leucocytes dans ledit sang dudit patient au cours du temps peut être découvert.
